# FASCICULE DE BREVET EUROPEEN

(11) **EP 0 649 829 B1**
(45) Date de publication et mention de la délivrance du brevet: **20.01.1999**
(21) Numéro de dépôt: 94870163.6
(22) Date de dépôt: 14.10.1994
(51) Int. Cl.: C07C 41/06, C07C 43/04, C10L 1/02

(54) **Procédé de production d'éthers de glycérol**
Verfahren zur Herstellung von Glyzerinethern
Process for the production of glycerol ethers

(30) Priorité: 15.10.1993 BE 9301097
(43) Date de publication de la demande: 26.04.1995
(73) Titulaire: FINA RESEARCH S.A., B-7181 Seneffe (BE)
(72) Inventeur: Dewattines, Carine, B-1870 Meise (BE); Hinnekens, Hervé, B-9230 Massemen Wetteren (BE)

(56) Documents cités:
- EP-A- 0 055 045
- EP-A- 0 407 841
- WO-A-94/01389
- US-A- 4 605 787
- US-A- 4 814 519
- US-A- 5 225 609
- CHEMICAL ABSTRACTS, vol. 97, no. 9, 30 Août 1982, Columbus, Ohio, US; abstract no. 71939b, page 593 ;colonne 1 ; & CS-A-190 755 (V MACHO ET AL)

## Description

La présente invention est relative à un procédé de production en une étape de tertioalkyl éthers de glycérol. Elle concerne également l'utilisation de ces éthers en tant que molécules biodégradables substituts des produits pétroliers.

Il est connu de préparer des éthers par réaction d'oléfines et d'alcools en présence de catalyseurs. Ainsi,il est déjà connu de faire réagir les oléfines, éventuellement en mélange avec des hydrocarbures paraffiniques, avec des alcools primaires ou secondaires en présence de catalyseurs acides pour donner des alkyl-éthers. En tant que catalyseurs acides, on utilise par exemple des acides minéraux comme l'acide sulfurique, ou des acides organiques comme les acides p-toluène-sulfoniques, ou les échangeurs de cations acides porteurs de groupes acide sulfonique à base de polymères aromatiques vinyliques réticulés (DE-A-1224294). On prépare également des alkyl éthers tertiaires au départ d'une oléfine tertiaire et d'un alcool, par exemple le méthyl tertiobutyl éther (MTBE) au départ de méthanol et d'isobutène, en présence de catalyseurs tels que les acides hétéropolytungstiques ou molybdiques, les alumines acidifiées ou différentes résines échangeuses d'ion.

Le brevet DD-A-133661 décrit un procédé pour la production d'un éther qui comprend la mise en contact d'une oléfine et d'un alcool avec une zéolite (faujasite, mordénite).

Le brevet EP-B-55045 décrit un procédé dans lequel intervient une zéolite ayant un rapport XO₂/Y₂O₃ égal ou supérieur à 10, dans laquelle X est un atome de silicium et ou de germanium et Y est un ou plusieurs atomes parmi l'aluminium, le fer, le chrome, le vanadium, le molybdène, l'arsenic, le manganèse, le gallium ou le bore, la zéolite étant principalement sous forme protonée, pour produire des éthers à partir d'oléfines et d'alcools primaires et secondaires.

La demande de brevet EP-A-407841 décrit la réaction d'oléfines tertiaires C₄-C₇ avec des alcools di- à hexahydriques pour produire des éthers, les catalyseurs étant sous forme acide.

L'utilisation d'un catalyseur comprenant une zéolite et un oxyde réfractaire non acide (comprenant un métal du groupe IV A et/ou IV B de la table périodique des éléments) est décrite dans la demande de brevet WO-A-9008120 relative à un procédé de conversion d'une oléfine en alcool et/ou éther par la réaction de l'oléfine avec de l'eau et/ou un alcool.

Le brevet US-A-3482952 concerne un procédé de production de carburant à haut indice d'octane, dans lequel des oléfines tertiaires C₄-C₆ et des alcools (C₁-C₆, primaires ou secondaires, mono ou polyfonctionnels) sont mis au contact de catalyseurs d'éthérification tels que des matériaux carbonés contenant des groupements -SO₃H.

Le brevet EP-B-198243 décrit l'utilisation de mélanges d'esters alkyliques d'acides gras et d'éthers de glycérol dans des carburants diesel.

Un inconvénient notable des procédés connus jusqu'à présent réside dans le fait qu'apparaissent des quantités importantes de sous-produits: oligomères d'oléfines, éthers dus à la condensation de l'alcool, polyéthers.

Il est en effet connu que les oléfines se convertissent en présence de catalyseurs acides en dimères, trimères et oligomères plus élevés (Erdöl und Kohle 19, 497, 1966).

Par ailleurs, on a besoin d'un procédé catalytique efficace pour fabriquer avec un bon rendement des trialkyl éthers de glycérol, limitant ainsi le nombre de fonctions alcool libres. En effet, ces fonctions alcool sont connues pour être hydrophiles et donc défavorables lors de l'utilisation en tant que carburant.

Le document WO-A-9401389, publié le 20.01.94, divulgue la préparation de polyol alkyl éthers par réaction, en présence d'un catalyseur acide, de glycérol avec des oléfines R₁ R₂ CCH₂ (avec R₁ = alkyle en C₁₋₆ et R₂ = H ou alkyle en C₁₋₆. Les exemples mentionnent des acides sulfoniques et une résine échangeuse d'ions acide comme catalyseurs.

Le brevet U.S. 5225609 divulgue la production d'alkyl éther au départ d'un alcanol (particulièrement le méthanol) et d'isooléfine en présence de zéolite aluminosilicate cristalline ayant un rapport silice/alumine d'au moins 12, un indice de contrainte d'environ 1 à 12, et une activité de craquage acide plus grande que 20.

Le brevet U.S 4814519 décrit la production en deux étapes d'éthers à partir d'une charge contenant des oléfines en présence d'un acide de Lewis qui peut être un catalyseur silicate cristallin poreux.

La demanderesse a trouvé de manière inattendue que des tertioalkyl éthers de glycérol peuvent être formés en présence de certaines zéolites et dans des conditions telles que la réaction du glycérol et de l'oléfine ramifiée sur la double liaison se produit avec une grande sélectivité. Le procédé de la présente invention a également pour objet la production de tertioalkyl éthers de glycérol avec un bon rendement. Le procédé de la présente invention a également pour objet la production de tertioalkyl éthers de glycérol en limitant les sous-produits indésirables tels que les oligomères de l'oléfine par exemple. Le procédé de la présente invention a enfin pour objet la production de tertioalkyl éthers de glycérol en présence d'un catalyseur qui puisse être éliminé facilement.

La présente invention fournit un procédé pour produire des tertioalkyl éthers à partir d'une oléfine et de glycérol, lequel procédé comprend :
* prévoir une charge hydrocarbonée comprenant essentiellement au moins une oléfine ramifiée sur la double liaison, ayant de 4 à 12 atomes de carbone, du glycérol et une zéolite utilisée sous forme acide et ayant les caractéristiques suivantes :
   - un rapport atomique Si/Al supérieur à 5,
   - des pores d'une ouverture de taille supérieure à 0,7 nm, et
   - des réseaux tridimensionnels de canaux croisés
* mettre en contact la charge hydrocarbonée et le glycérol avec le solide catalytique dans des conditions efficaces pour former les éthers, les conditions comprenant:
   a) une pression de 0,3 à 7 MPa
   b) une température de 50 à 200° C
   c) un rapport molaire oléfine:glycérol compris entre 6:1 à 1:1
   d) un rapport pondéral catalyseur:glycérol compris entre 0,01 et 10%
* récupérer les tertioalkyl éthers.

Selon un mode de réalisation préféré, le glycérol réagit avec de l'isobutène en présence de zéolite Y ultrastable sous forme acide pour fournir des polyéthers tertiobutyliques de glycérol avec une sélectivité élevée.

Selon un autre mode d'exécution préféré, le glycérol réagit avec de l'isobutène en présence de zéolite β, désaluminisée et sous forme acide, pour fournir des mono- et di-éthers tertiobutyliques de glycérol avec une sélectivité élevée.

Le glycérol utilisé dans la présente invention est généralement un sous-produit abondant de la synthèse des esters d'acides gras.

Dans le procédé conforme à l'invention, on utilise des oléfines ramifiées sur la double liaison. A titre d'exemple, on mentionnera les isooléfines ayant de 4 à 12, de préférence 4 à 8, tout particulièrement 4 à 6 atomes de carbone, comme l'isobutylène qui est préférentiellement utilisé.

Ces oléfines peuvent être mises en jeu sous une forme pure, sous forme de mélange d'oléfines ramifiées sur la double liaison ou sous forme d'un mélange avec un ou plusieurs hydrocarbures saturés. Par exemple, on peut employer dans le procédé conforme à l'invention une fraction, produite dans une raffinerie par des procédés de craquage, contenant une des isooléfines ramifiées sur la double liaison, citées plus haut, à côté d'hydrocarbures saturés et éventuellement de gaz inertes comme l'azote, l'hydrogène ou le dioxyde de carbone. Ainsi, l'industrie pétrochimique produit des courants d'oléfines dans la gamme C₂ - C₇, et la conversion de ces courants ou de fraction de ceux-ci en éthers peut fournir des produits utiles en tant que substituts de produits pétroliers.

En outre, il est possible d'employer, dans le procédé conforme à l'invention, des mélanges ou des fractions de craquage qui contiennent 2 ou plusieurs oléfines ramifiées sur la double liaison, éventuellement à côté de paraffines et/ou des substances inertes citées, et de séparer les éthers obtenus, après l'exécution de la réaction conforme à l'invention, par des mesures appropriées, par exemple par distillation.

Les oléfines et leurs mélanges utiles dans le procédé de la présente invention incluent les mono ou dioléfines.

Pour le procédé conforme à l'invention, on utilise une zéolite acide à large pores dont le rapport atomique Si/Al est supérieur à 5. De préférence, le rapport atomique Si/Al est compris entre 8,5 et 40, de préférence environ 28. Par zéolite à larges pores, on entend une zéolite dont les pores ont une ouverture de taille supérieure à 0,7 nm. De plus, il est essentiel que la zéolite utilisée ait une structure comportant des réseaux tridimensionnels de canaux croisés, comme le montrent les exemples comparatifs.

Selon un mode préféré d'exécution de la présente invention, on utilise une zéolite Y ultrastable sous forme acide en raison de son activité élevée et de sa forte sélectivité pour la production de tritertioalkyl éthers de glycérol.

Selon un autre mode préféré d'exécution de la présente invention, on utilise une zéolite β désaluminisée sous forme acide en raison de son activité élevée et de sa forte sélectivité pour la production de mono- et di- tertioalkyl éthers de glycérol.

De préférence, les zéolites ont un paramètre d'unité cellulaire compris entre 2,425 et 2,450 nm.

Le catalyseur est de préférence présent dans le mélange dans des quantités variant de 1 à 5% en poids par rapport au glycérol.

Les températures de la réaction varient de 50 à 200°C, de préférence de 80 à 120° C.

La réaction est exécutée sous une pression de 0,3 à 7 MPa, de préférence de 0,6 à 2,1 MPa.

L'oléfine ramifiée sur la double liaison est utilisée dans un rapport molaire envers le glycérol de 6:1 à 1:1, de préférence de 5:1 à 1:1.

Lorsqu'on utilise plusieurs oléfines ramifiées sur la double liaison, le rapport se réfère au nombre total de moles de ces oléfines.

Dans le cas de la préparation de monoalkylglycéroléther, le rapport oléfine:glycérol sera environ 1:1. Mais si l'on désire préparer préférentiellement les di/tri alkyléthers de glycérol, le milieu sera riche en oléfine et le rapport oléfine:glycérol sera supérieur à 1:1.

Le procédé de l'invention peut être exécuté de la façon suivante : dans un réacteur fermé, on introduit le glycérol mélangé au catalyseur dans un rapport de 0,01 à 10 parties en poids de catalyseur par parties en poids de glycérol; le réacteur est ensuite purgé à l'azote et mis sous pression avec la charge hydrocarbonée dans un rapport choisi en fonction de la sélectivité désirée. Le mélange est alors chauffé sous agitation pendant 3 à 6 heures, de façon que l'éthérification soit achevée. Le réacteur est alors refroidi et le produit récupéré sous forme de liquide incolore. Si on le souhaite, on peut aisément séparer ensuite les mono-, di- et tri-éthers.

Les éthers de glycérol ainsi formés sont biodégradables et peuvent être utilisés comme substituts de produits pétroliers dans de nombreuses applications.

A titre d'exemples et de manière non limitative, ils peuvent être utilisés comme améliorants de l'indice d'octane des carburants mais aussi comme substituts du MTBE lorsque l'on désire réduire la volatilité du carburant.

Ils peuvent également servir à abaisser le point d'écoulement dans les produits pétroliers en général.

De même, les éthers de glycérol sont de bons solvants notamment pour des laques compte tenu de leurs propriétés dissolvantes vis-à-vis des nitrocelluloses, gommes, résines, huiles et plastifiants.

Les exemples suivants sont donnés afin de mieux illustrer le procédé de la présente invention, mais sans pour autant en limiter la portée.

### Exemple 1

Préparation du glycérol mono-t-butyléther.
Dans un réacteur fermé de 600cc équipé de contrôles de chauffage, agitation et température, on a introduit un mélange de 145,6 g de glycérol (1,58 moles) et de 4,684 g de zéolite Y ultrastable ayant un rapport atomique Si/Al de 20 et un paramètre d'unité cellulaire de 2,428 nm. Le réacteur a ensuite été purgé à l'azote, mis sous une pression de 0,6895 MPa avec 88,6 g d'isobutylène (1,58 moles) et chauffé à 90°C en agitant. Après 4 h de réaction, le réacteur a été refroidi et le produit récupéré sous forme de liquide incolore.
L'analyse par chromatographie en phase gazeuse a montré la présence de

| | (% en poids) |
|---|---|
| glycérol mono-t-butyléther | 78,00 |
| glycérol di-t-butyléther | 13,865 |
| glycérol tri-t-butyléther | 8,135 |

### Exemple 2

Préparation du glycérol tri-t-butyléther.
Dans un réacteur fermé de 600cc équipé de contrôles de chauffage, agitation et température, on a introduit un mélange de 109,1 g de glycérol (1,19 moles) et de 7,51 g de zéolite identique à celle de l'exemple 1. Le réacteur a ensuite été purgé à l'azote, mis sous une pression de 0,6895 MPa avec 267,6 g d'isobutylène (4,78 moles) et chauffé à 90°C en agitant.

Après 4 h de réaction, le réacteur a été refroidi et le produit récupéré sous forme de liquide incolore.
L'analyse par chromatographie en phase gazeuse a montré la présence de

| | (% en poids). |
|---|---|
| glycérol mono-t-butyléther | 0,9 |
| glycérol di-t-butyléther | 11 |
| glycérol tri-t-butyléther | 60 |
| isobutylène | 29,1 |

### Exemples 3 et 4 et exemples comparatifs

On a fait réagir 1 môle de glycérol avec 3 môles d'isobutylène en présence de 1 g de divers catalyseurs acides, à 1,5 MPa et 88°C pendant 5 heures. Les catalyseurs et les résultats sont repris dans le tableau 1, en mol% sauf indication contraire.

Comme le montrent les exemples comparatifs (Tableau 1), l'utilisation d'une zéolite selon l'invention est essentielle pour obtenir à la fois de bons rendements, de faibles productions de produits indésirables, et l'élimination aisée du catalyseur :
- l'acide toluène sulfonique donne les meilleurs résultats, mais il n'est pas éliminé facilement par filtration;
- l'acide phosphotungstique donne les résultats équivalents, mais n'est pas éliminé facilement par filtration;
- le sel phosphotungstique et la résine acide donnent d'importantes quantités d'oligomères d'isobutylène;
- les autres zéolites ont un rendement faible ou nul.

### Exemple 5

Les résultats suivants ont été obtenus avec des zéolites Y après réaction d'isobutylène avec du glycérol (rapport molaire 3:1) pendant 24 heures à 80°C et 1,5 MPa :

| Rapport Si/Al | % conversion glycérol | monoéthers (mol %) | diéthers (mol %) | triéthers (mol %) |
|---|---|---|---|---|
| 8,5 | 83,3 | 16,8 | 58,8 | 1,9 |
| 14,5 | 99,4 | 20,5 | 63,1 | 9,7 |
| 28 | 98,7 | 21,7 | 59,8 | 8,6 |
| 40 | 99,2 | 19,9 | 61,5 | 11,2 |

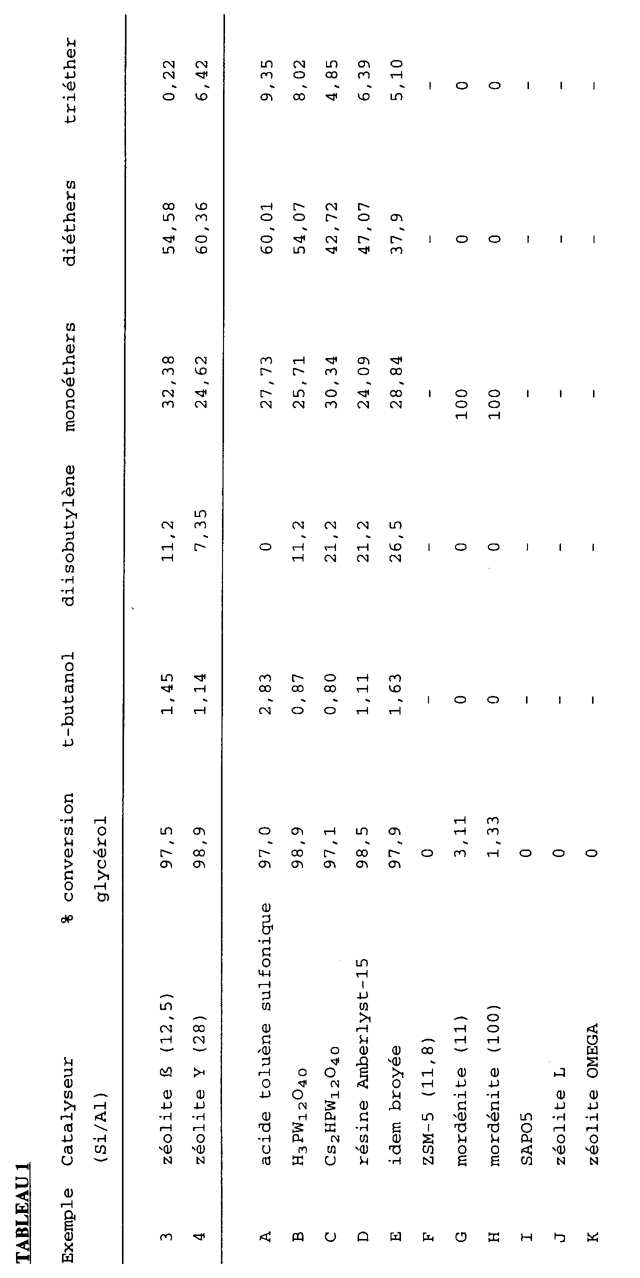

## Revendications

1. Procédé de production de tertioalkyl éthers de glycérol lequel procédé comprend:
* prévoir une charge hydrocarbonée comprenant une ou plusieurs oléfines ramifiées sur la double liaison, ayant de 4 à 12 atomes de carbone, du glycérol et une zéolite utilisée sous forme acide et ayant les caractéristiques suivantes :
- un rapport atomique Si/Al supérieur à 5,
- des pores d'une ouverture de taille supérieure à 0,7 nm
- des réseaux tridimensionnels de canaux croisés
* mettre en contact la charge hydrocarbonée et le glycérol avec le solide catalytique dans des conditions efficaces pour former les éthers, les conditions comprenant :
a) une pression de 0,3 à 7 MPa
b) une température de 50 à 200° C
c) un rapport molaire oléfine:glycérol compris entre 6:1 à 1:1
d) un rapport pondéral catalyseur:glycérol compris entre 0,01 et 10%
* récupérer les tertioalkyl éthers.

2. Procédé selon la revendication 1, caractérisé en ce que la zéolite a un rapport atomique Si/Al compris entre 8,5 et 40, de préférence environ 28.

3. Procédé selon l'une des revendications 1 ou 2, caractérisé en ce que la zéolite est une zéolite Y ultrastable.

4. Procédé selon l'une des revendications 1 ou 2, caractérisé en ce que la zéolite est une zéolite β.

5. Procédé selon l'une des revendications 1 à 4, caractérisé en ce que la zéolite a un paramètre d'unité cellulaire compris entre 2,425 et 2,450 nm.

6. Procédé selon l'une des revendications 1 à 5, caractérisé en ce que l'oléfine est l'isobutylène.

7. Procédé selon l'une des revendications 1 à 6, caractérisé en ce que l'éthérification est réalisée sous des pressions de 0,6 à 2,1 MPa.

8. Procédé selon l'une des revendications 1 à 7, caractérisé en ce que le rapport molaire oléfine : glycérol est compris entre 5:1 à 1:1.

9. Procédé selon l'une de revendications 1 à 8, caractérisé en ce que l'éthérification est réalisée à une température de 80 à 120°C.

## Claims

1. Process for the production of tertiary alkyl glycerol ethers, said process comprising:
* providision of a hydrocarbonated charge comprising one or more olefins branched on the double bond, having 4 to 12 carbon atoms, glycerol and a zeolite used in acid form and having the following characteristics:
- an atomic ratio of Si/Al greater than 5
- pores with an aperture size greater than 0.7 nm
- three-dimensional networks of crossed channels
* bringing into contact the hydrocarbonated charge and the glycerol with the catalytic solid in effective conditions for forming ethers, which conditions comprising:
a) a pressure from 0.3 to 7 MPa
b) a temperature from 50° to 200°C
c) a molar ratio of olefin to glycerol in the range of between 6:1 and 1:1
d) a gravimetric ratio of catalyst to glycerol in the range of between 0.01 and 10%
* recovery of the tertiary alkyl ethers.

2. Process according to Claim 1, characterised in that the zeolite has an atomic ratio of Si/Al in the range of between 8.5 and 40, preferably approximately 28.

3. Process according to one of Claims 1 or 2, characterised in that the zeolite is an ultrastable Y zeolite.

4. Process according to one of Claims 1 or 2, characterised in that the zeolite is a β zeolite.

5. Process according to one of Claims 1 to 4, characterized in that the zeolite has a cellular unit parameter in the range of between 2.425 and 2.450 nm.

6. Process according to one of Claims 1 to 5, characterized in that the olefin is isobutylene.

7. Process according to one of Claims 1 to 6, characterized in that etherification is achieved at pressures of 0.6 to 2.1 MPa.

8. Process according to one of Claims 1 to 7, characterized in that the molar ratio of olefin to glycerol is in the range of between 5:1 and 1:1.

9. Process according to one of Claims 1 to 8, characterized in that etherification is achieved at a temperature of 80° to 120°C.

## Patentansprüche

1. Verfahren zur Herstellung von Tertiäralkylethern von Glycerol, wobei das verfahren umfaßt:
* Vorsehen einer Kohlenwasserstoffladung, enthaltend ein oder mehrere an der Doppelbindung verzweigte Olefine, 4 bis 12 Kohlenstoffatome aufweisend, Glycerol und einen unter saurer Form eingesetzten Zeolithen und welcher die folgenden charakteristischen Eigenschaften hat;
- ein Atomverhältnis Si/Al oberhalb von 5,
- Poren mit einer Öffnungsgröße oberhalb von 0,7 nm,
- dreidimensionale Netzwerke mit gekreuzten Kanälen
* In Berührung bringen der Kohlenwasserstoffladung und des Glycerols mit dem katalytischen Feststoff unter wirksamen Bedingungen, um die Ether Zu bilden, wobei die Bedingungen umfassen:
a) einen Druck von 0,3 bis 7 MPa
b) eine Temperatur von 50 bis 200°C
c) ein Molverhältnis von Olefin:Glycerol, das zwischen 6:1 bis 1:1 enthalten ist
d) ein Gewichtsverhältnis Katalysator:Glycerol, das zwischen 0,01 und 10% enthalten ist
* Gewinnen der Tertiäralkylether.

2. Verfahren nach Anspruch 1, dadurch gekennzeichnet, daß der Zeolith ein Atomverhältnis Si/Al hat, das zwischen 8,5 und 40 enthalten ist, vorzugsweise ungefähr 28 ist.

3. Verfahren nach einem der Ansprüche 1 oder 2, dadurch gekennzeichnet, daß der Zeolith ein ultrastabiler Zeolith Y ist.

4. Verfahren nach einem der Ansprüche 1 oder 2, dadurch gekennzeichnet, daß der Zeolith ein Zeolith β ist.

5. Verfahren nach einem der Ansprüche 1 bis 4, dadurch gekennzeichnet, daß der Zeolith einen Zelleinheitsparameter hat, der zwischen 2,425 und 2,450 nm enthalten ist.

6. Verfahren nach einem der Ansprüche 1 bis 5, dadurch gekennzeichnet, daß das Olefin das Isobutylen ist.

7. Verfahren nach einem der Ansprüche 1 bis 6, dadurch gekennzeichnet, daß die Verätherung unter Drücken von 0,6 bis 2,1 MPa realisiert wird.

8. Verfahren nach einem der Ansprüche 1 bis 7, dadurch gekennzeichnet, daß das Molverhältnis Olefin:Glycerol zwischen 5:1 bis 1:1 enthalten ist.

9. Verfahren nach einem der Ansprüche 1 bis 8, dadurch gekennzeichnet, daß die Verätherung bei einer Temperatur von 80 bis 120°C durchgeführt wird.
